(19) Europäisches Patentamt
European Patent Office
Office européen des brevets

(11) **EP 0 603 967 B1**

(12) **FASCICULE DE BREVET EUROPEEN**

(45) Date de publication et mention
de la délivrance du brevet:
**13.09.2000 Bulletin 2000/37**

(51) Int. Cl.⁷: **A61B 8/06**, G01S 15/89,
G01S 15/58

(21) Numéro de dépôt: **93203600.7**

(22) Date de dépôt: **21.12.1993**

(54) **Dispositif et procédé de mesure d'élasticité d'une artère par échographie ultrasonore**

Gerät und Verfahren zur Elastizitätsmessung einer Arterie mittels Ultraschall-Echographie

Means and process to measure the elasticity of an artery by ultrasonic echography

(84) Etats contractants désignés:
**DE FR GB**

(30) Priorité: **22.12.1992 FR 9215506**

(43) Date de publication de la demande:
**29.06.1994 Bulletin 1994/26**

(73) Titulaire:
**Koninklijke Philips Electronics N.V.
5621 BA Eindhoven (NL)**

(72) Inventeur: **Bonnefous, Odile
F-75008 Paris (FR)**

(74) Mandataire:
**Charpail, François et al
Société Civile S.P.I.D.
156, Boulevard Haussmann
75008 Paris (FR)**

(56) Documents cités:
**EP-A- 0 075 284        EP-A- 0 127 157
GB-A- 2 156 985**

EP 0 603 967 B1

**Description**

La présente invention concerne un dispositif et un procédé de mesure de paramètres physiologiques d'une artère sous l'effet de la pression sanguine P comportant, dans un échographe ultrasonore utilisé en profilomètre (mode M), et muni de moyens d'émission-réception incorporant un dispositif de formation de voies en réception, un premier ensemble de mesure et de calcul du débit sanguin instantané $Q(t)$, de la variation de rayon instantanée $\Delta r(t)$ de ladite artère et de son rayon moyen $r_o$ selon une première ligne de tir A, à partir de mesures de profondeurs d'exploration z pour le sang ou les parois de l'artère obtenues avec une précision de l'ordre du micron.

La mécanique circulatoire est étudiée depuis très longtemps et très bien décrite de nos jours. Les données ont été collectées par des mesures in vivo et in vitro sur des modèles animaux ou humains mais la plupart du temps de manière invasive ou traumatique. Cependant, les nouveaux outils ultrasonores peuvent permettre des mesures in vivo non invasives comme les techniques Doppler pulsé par exemple.

Les procédés de corrélation temporelle utilisés dans la technique du CVI (Colour Velocity Imaging en anglais) permettent de définir des mesures et des outils de mesure adaptés à la caractérisation mécanique des artères. L'art antérieur d' arrière plan technologique dans ce domaine consiste surtout en ouvrages scientifiques qui décrivent la mécanique circulatoire ainsi que les moyens précédemment utilisés pour son étude, tel par exemple l'ouvrage : Mc Donald's Blood flow in Arteries, par W. W. Nichols et M. F. O'Rourke, édité chez Edward Arnold, Londres.

Un tel dispositif conforme à celui indiqué au premier paragraphe est connu notamment de la demande de brevet européen n° 0 458 384 A1 au nom de la demanderesse (PHF90/536) incorporée par référence au présent texte. Ce dispositif s'applique de préférence à l'exploration échographique d' écoulements sanguins dans les vaisseaux, en particulier à la mesure et à la visualisation, aux fins de diagnostic, de paramètres physiologiques caractéristiques de ces écoulements.

Une réalisation du dispositif selon la demande précitée comprend une première unité de mesure par échographie ultrasonore de la vitesse $V(t,z)$ de l'écoulement sanguin en fonction du temps t et de la profondeur d'exploration z, la mesure de la vitesse $V(t,z)$ étant indépendante de l'onde ultrasonore utilisée et une mémoire de stockage des échantillons de vitesse $V(t,z)$. Cette réalisation est remarquable en ce qu'elle comporte en outre en combinaison des premiers circuits de calcul du débit instantané $Q(t)$ déduit desdits échantillons de vitesse $V(t,z)$ ainsi qu'une deuxième unité de mesure de la vitesse radiale $V_1(t,z)$ et $V_2(t,z)$ de déplacement des deux parois de vaisseau qui bornent diamétralement ledit écoulement sanguin, des mémoires de stockage des valeurs de vitesse $V_1(t,z)$ et $V_2(t,z)$, des deuxièmes circuits de calcul comprenant un circuit de calcul de l'énergie locale non débarrassée des échos fixes $E_2(t,z)$, suivi d'un circuit de calcul d'épaisseurs respectives $d_1 = z_4 - z_3$ et $d_2 = z_6 - z_5$ desdites parois constitué par un détecteur de seuil de valeur $E'_0$ pour la détermination des valeurs $z_3$, $z_4$, $z_5$, $z_6$, deux circuits de calcul des vitesses moyennes respectives $\hat{V}_1(t_0)$ et $\hat{V}_2(t_0)$ desdites parois pour chaque valeur de temps $t_0$ constitués par un additionneur donnant $\Sigma_{d1}V_1(t,z)$ (respectivement $\Sigma_{d2}V_2(t,z)$) et par un diviseur par $M_1$ (respectivement $M_2$), $M_1$ et $M_2$ étant le nombre d'échantillons de mesure sur le segment $[z_3, z_4]$ (respectivement $[z_5, z_6]$), un circuit de calcul du déplacement de chaque paroi constitué par un additionneur donnant :

$$D_1(t) = \Sigma_t \hat{V}_1(t_0) \text{ et } D_2 = \Sigma_t \hat{V}_2(t_0),$$

et un circuit de calcul du déplacement symétrique des parois constitué par un soustracteur et un diviseur par 2 donnant sous forme d'échantillons temporels la variation de rayon instantanée dudit vaisseau $\Delta r(t) = (D_2(t) - D_1(t))/2$ et des moyens de visualisation des courbes $Q(t)$ et $\Delta r(t)$ en fonction du temps t.

La connaissance simultanée du débit $Q(t)$ et de la variation du rayon du vaisseau $\Delta r(t)$ d'une artère notamment, permet d'effectuer divers calculs et se prête à plusieurs représentations fort utiles pour le praticien. En effet, on peut considérer que la fonction $\Delta r(t)$ est une image de la pression $P(t)$ qui règne dans le vaisseau, en ce sens que, la dilatation du vaisseau étant une fonction directe de la pression, les phases de croissance et de décroissance de ces deux fonctions sont les mêmes au cours du cycle cardiaque. Dès lors, il est loisible d'assimiler $P(t)$ à $\Delta r(t)$, en première approximation, à un facteur de proportionnalité près.

Un mode de représentation particulièrement intéressant du débit et de la variation de pression combinés consiste à calculer et à visualiser la courbe du cycle cardiaque, paramétrée en temps et rebouclée sur elle-même, constituée par les points obtenus en portant en ordonnées les valeurs-échantillons de la fonction $\Delta r(t)$ et en abscisses les valeurs-échantillons de la fonction du débit instantané $Q(t)$, le produit $Q \times \Delta r$ qui est une portion du plan de visualisation étant alors homogène à une puissance en première approximation.

De là on peut déduire notamment, à partir de la mesure de la surface du cycle et de la surface sous-tendue par le cycle, le calcul de l'efficacité artérielle pour la tranche d'épaisseur axiale dx du vaisseau analysé.

Le calcul d'efficacité qui s'exprime par des rapports de puissance et, sur la durée d'un cycle ou d'une portion de cycle par des rapports d'énergie, reflète directement l'énergie perdue dans la portion de vaisseau analysée pendant un cycle et donc la rugosité et/ou l'étranglement locaux de la paroi du vaisseau, ce qui est, pour le praticien, d'une aide précieuse pour la détection des sténoses. De plus, le mode de représentation en boucle précité révèle, pour chaque type d'artère, une forme très caractéristique assimilable à une "signature" et l'on peut prévoir que, l'expérience aidant, telle ou telle anomalie de forme pourra être interprétée par le praticien comme révélatrice de telle ou telle pathologie du coeur et/ou de l'artère analysée.

Pour accéder au mode de représentation indiqué cidessus et à certains autres, une grande précision est nécessaire dans la détermination des fonctions Q(t) et Δr(t).

Parmi les échographes ultrasonores permettant d'obtenir cette grande précision et notamment de fournir, en amont, une mesure de vitesses sanguines indépendantes de la fréquence ultrasonore, on peut citer ceux qui fonctionnent sur le principe de la corrélation temporelle déjà décrite dans la demande de brevet européen n° 0 225 667 au nom de la demanderesse (PHF85/593), et dont l'unité de mesure de la vitesse d'écoulement comprend un circuit d'intercorrélation délivrant à partir de deux échos successifs des valeurs de fonction de corrélation, et un circuit de multiplexageinterpolation fournissant à partir desdites valeurs des fonctions de corrélation une estimée de la vitesse V(t,z).

A partir d'un état de l'art tel que celui brossé ci-dessus, on peut souhaiter en arriver à la détermination de paramètres en fonction du temps très caractéristiques des phénomènes hydrodynamiques qui se produisent dans une artère. Plus précisément, on se propose de déterminer l'élasticité $\gamma$ et la pression P en un point quelconque d'une artère, en premier lieu la valeur moyenne $\gamma_0$ et $P_0$ de ces paramètres sur la durée d'un cycle cardiaque, et si possible leurs variations au cours du temps, soit les fonctions $\gamma(t)$ et P(t). L'élasticité d'une artère, que l'on peut définir par la relation différentielle : $\gamma = dr/dP$ , est aussi appelée compliance (en anglais notamment). On peut en trouver une description dans l'article "Mesure de la compliance artérielle" par Georges Nicod, EPFL (Ecole Polytechnique Fédérale de Lausanne), Presse et information, en liaison avec les variations de pression dans l'artère. Dans cet article est aussi décrite une méthode de détermination de la compliance rapportée à la pression. La mesure de la pression artérielle en continu s'effectue au moyen d'un photopléthysmographe, appareil qui mesure les différences de pression à l'extrémité d'un doigt. Par ailleurs, le diamètre de l'artère au point d'analyse est quant à lui déterminé par un transducteur ultrasonore, en mode écho pulsé (ou suiveur d'écho). L'application d'un déphasage approprié, rapporté au cycle cardiaque, entre les capteurs de pression et de diamètre, permet de recaler entre elles les deux courbes obtenues et d'en faire le rapport. Cette méthode permet d'obtenir une courbe de compliance qui peut s'exprimer en pour cents d'accroissement du diamètre par unité de pression, rapportés à la pression. Il est ainsi possible de caractériser le comportement d'un point quelconque d'une artère et d'en déduire l'élasticité de cette dernière en ce point. Cependant cette détermination reste approximative dans la mesure où la détermination de la pression sanguine n'est pas effectuée localement mais toujours au même endroit, à l'extrémité d'un doigt.

Un autre article, par MOOSER et al, "Diameter of peripheral arteries in cardiac cycle", Journal of Hypertension 1988, 6 (suppl.4) : S179-S181, publié en Suisse, montre comment mesurer in vivo, avec précision, le diamètre instantané d'une artère, de manière non invasive.

Du brevet GB-A-2 156 985 est connu un échographe ultrasonore qui, pour la détermination de l'élasticité et de la pression dans un vaisseau sanguin, met en oeuvre l'appréciation de la vitesse de propagation de l'onde de pression artérielle le long du vaisseau à partir de deux tirs effectués par l'échographe dans le vaisseau.

L'invention se propose de déterminer l'élasticité (compliance) d'une artère à partir de mesures échographiques intégralement effectuées à un endroit prédéterminé de l'artère analysée.

Un autre but de l'invention est de déterminer la compliance d'une artère à un endroit prédéterminé de cette dernière avec une précision accrue.

Ces buts sont atteints et les inconvénients de l'art antérieur sont atténués ou supprimés grâce au fait que le dispositif de mesure de paramètres physiologiques défini en préambule est remarquable en ce qu'il comporte en outre, pour la détermination de l'élasticité instantanée $\gamma(t)$ de ladite artère et de la pression instantanée P(t) à l'intérieur de cette dernière, par une méthode autre que celles mettant en oeuvre l'appréciation de la vitesse de propagation de l'onde de pression artérielle le long de l'artère, un deuxième ensemble de mesures et de calcul par échographie ultrasonore des paramètres instantanés Q' (t) et Δr' (t), simultanés ou recalés dans le temps par rapport aux valeurs instantanées Q(t) et Δr(t), selon une deuxième ligne de tir B située à une distance d'écart e de la première ligne de tir A, comprise entre quelques dixièmes de millimètres et quelques millimètres, telle qu'elle soit assimilable à une mesure différentielle, les deux lignes de tir A et B traversant l'artère suivant un de ses plans méridiens, la distance d'écart e étant prise dans la direction de l'axe de l'artère, des premiers et deuxièmes moyens de mémorisation pour le stockage d'échantillons numériques de signaux relatifs aux flux sanguins et de signaux relatifs aux parois de l' artère étant prévus en sortie desdits premier et deuxième ensembles de mesure, ainsi que des moyens de calcul effectués à partir desdits échantillons numériques de signaux.

Partant de l'équation fondamentale :

$$R\,Q\,(t) + L\,\frac{dQ(t)}{dt} + \frac{\Delta P}{\Delta x} = -\,\frac{1}{\gamma}\,\frac{\partial r}{\partial x} \qquad (1)$$

dans laquelle :

R : résistance hydrodynamique du sang,

$$L = L_0 = \rho/\Pi r_0^2$$

avec $\rho$ : masse volumique du sang,

$$\Delta P/\Delta x = RQ_0$$

soit le gradient de pression sanguine le long de l'artère,
et où $\partial r/\partial x$ mesurable, est identifiée à la variation instantanée de rayon interne de l'artère sur la distance d'écart e,

il est possible de calculer de préférence par application de la méthode des moindres carrés, la valeur de la compliance $\gamma$ notamment.

Dans un premier temps, sont calculées les valeurs moyennes $R_0$, $R_0Q_0$ et $\gamma_0$, sur un cycle cardiaque, puis, par itérations successives, R(t) et $\gamma$(t) sous forme de fonctions constantes par morceaux, soit quelques échantillons des deux

fonctions au cours de ce cycle. Par ailleurs, la pression P(t) peut aussi être calculée par intégration de l'équation aux dérivées partielles :

$$\frac{\partial P(t)}{\partial t} = - \frac{1}{\gamma(t)} \frac{\partial r(t)}{\partial t} \tag{2}$$

Cette fonction P(t) est obtenue à une constante additive près mais on verra plus loin qu'il est possible de connaître exactement la valeur correcte de P(t) par détermination de la valeur moyenne $P_0$ au cours du cycle cardiaque.

A noter que les calculs précités n'ont de sens, d'un point de vue physique, que si la précision de mesure est suffisante. Pour cela, il faut que l'échographe ultrasonore utilisé soit apte à mesurer des profondeurs d'exploration z avec une précision de l'ordre du micron.

A noter aussi que ces calculs sont originaux en ce qu'ils mettent en oeuvre des mesures différentielles et des mesures de dérivées instantanées, et qu'ils n'utilisent pas, pour l'appréciation de la compliance, la détermination préalable de la vitesse de propagation de l'onde de pression le long de l'artère, comme c'est classiquement le cas, dans l'art connu, ainsi que décrit par exemple dans l'ouvrage : Biomécanique circulatoire, par R. COMOLET, publié par MASSON, 1984, pages 164 et 165 notamment.

Selon un premier mode de réalisation préféré, le dispositif de l'invention est remarquable en ce qu'il comporte des moyens d'émission-réception d'un échographe ultrasonore fonctionnant selon le principe de la corrélation temporelle et destiné à la fourniture d'images ultrasonores en mode bidimensionnel (mode 2D), dont la détection, limitée à deux lignes de tir voisines A et B séparées par la distance d'écart e, est effectuée paire par paire de points homologues des deux lignes de tir, ainsi que des moyens de recalage de phase des signaux de réception desdites deux lignes de tir voisines intégrés dans lesdits moyens de calcul.

Le mode bidimensionnel ou mode 2D, bien connu en échographie ultrasonore pour la fourniture d'images selon des plans de coupe du patient examiné effectue, au moyen d'un balayage électronique pour l'émission-réception de transducteurs élémentaires adjacents de la sonde, des lignes de l'image, qui se succèdent dans le temps, à raison d'un tir par ligne. Pour la mise en oeuvre de l'invention, la fourniture de deux lignes voisines (tirs A et B) suffit, ces deux lignes pouvant être séparées par le pas d'espacement des lignes de l'image du mode 2D, soit par exemple $e_0 = 0,45$ mm, soit plusieurs fois ce pas, soit par exemple $e_0 = 4,5$ mm pour un saut de 9 lignes de l'image. Le décalage temporel entre les deux lignes, lié à leur décalage spatial, doit être exactement compensé pour la mesure d'échantillons de certaines fonctions, telle la fonction du temps $\partial r/\partial x$ notamment. Cette compensation se traduit par une remise en phase des signaux reçus des tirs A et B, qui se déduit aisément de la durée $\tau$, connue avec précision, séparant les tirs A et B.

Selon un deuxième mode de réalisation préféré, le dispositif de l'invention est remarquable en ce qu'il est constitué par un échographe ultrasonore fonctionnant selon le principe de la corrélation temporelle, utilisé en profilomètre, dans lequel les moyens d'émission sont adaptés pour émettre un faisceau ultrasonore plus puissant et légèrement défocalisé par rapport à ceux d'un profilomètre conventionnel, et qui incorpore un dispositif de formation de voies en réception double pour fournir en parallèle et simultanément, en sortie des deux dispositifs de formation de voies, les signaux de deux lignes de tir A et B séparées par la distance d'écart e.

Dans cette version dont le fonctionnement peut être qualifié de double mode M, le recalage en phase des signaux reçus des deux tirs simultanés A et B n'est plus nécessaire.

Afin d'obtenir la précision requise, sur les deux fonctions r(t) physiquement séparées par la faible distance e notamment, on préférera l'utilisation d'échographes basés sur le principe de la corrélation temporelle aux systèmes Doppler conventionnels du fait de la mauvaise résolution axiale de ces derniers. Un échographe à corrélation temporelle est décrit pas exemple dans la demande de brevet européen précitée n° 0 225 667 au nom de la demanderesse (PHF85/593).

Les signaux d'écho des tirs A et B étant mémorisés sous forme d'échantillons temporels dans des mémoires, il est possible, après remise en phase éventuelle, en utilisant des moyens de calcul informatiques, de déterminer les fonctions périodiques $\gamma(t)$ et P(t).

Un premier procédé de mesure de l'élasticité $\gamma$ d'une artère sous l'effet de la pression sanguine P, au moyen d'un échographe ultrasonore apte à fournir le débit sanguin instantané Q(t), la variation de rayon instantanée $\Delta r(t)$ et le rayon moyen $r_0$ pour deux lignes de tir parallèles rapprochées A et B traversant l'artère suivant un de ses plans méridiens, pour la mise en oeuvre du dispositif selon les modes de réalisation décrits ci-dessus, lesdites valeurs Q(t), $\Delta r(t)$ et $r_0$ étant stockées dans lesdits premiers et deuxième moyens de mémorisation, comporte les étapes suivantes :

i - le calcul, à partir de l'une des deux fonctions Q(t),

Q'(t) de la valeur moyenne $Q_0$ et de la dérivée : $\frac{\partial Q(t)}{\partial t}$ de cette fonction.

ii - le calcul, à partir des valeurs instantanées $\Delta r(t)$, $\Delta r'(t)$ pour les lignes A et B de la valeur instantanée :

$$\delta r(t) = \Delta r(t) - \Delta r'(t)$$

iii - la détermination, à partir de la méthode des moindres carrés appliquée, sur un cycle cardiaque, à la relation :

$$\gamma \left[ R\, Q\, (t) + L\, \frac{\partial Q(t)}{\partial t} + \frac{\Delta P}{\Delta x} \right] = -\, \frac{\partial r(t)}{\partial x}$$

dans laquelle est effectuée l'identification :

$$L = \frac{\rho}{\Pi r_0^2}, \text{ et } \frac{\partial r(t)}{\partial x} = \frac{\delta r(t)}{e}$$

des valeurs moyennes des paramètres d'élasticité $\gamma$, de résistance hydrodynamique R et de gradient de pression $\Delta P/\Delta x$, soit $\gamma_0$, $R_0$ et $R_0 Q_0$ respectivement.

Un deuxième procédé de mesure de l'élasticité $\gamma$ d'une artère qui se déduit du premier procédé décrit ci-dessus est remarquable en ce que, pour la détermination par morceaux sur au moins un cycle cardiaque des fonctions $\gamma(t)$ et R(t), la méthode des moindres carrés est à nouveau appliquée par itérations successives sur des parties complémentaires du cycle cardiaque en nombre croissant, d'une itération à la suivante, en prenant, pour la première itération, deux demi cycles cardiaques (puis 4, 8, ..., parties de cycles pour les itérations suivantes), et la relation :

$$\gamma 0(1+g'\,(t)) \left[ R_0(1+R'(t))\, Q(t) + L\, \frac{\partial Q}{\partial t} + R_0\, Q_0 \right] = -\, \frac{\delta r(t)}{e},$$

ce qui fournit pour $\gamma$ et R des fonctions échantillonnées de quelques échantillons sur la durée du cycle cardiaque.

Pour ce deuxième procédé, les calculs à effectuer sont plus complexes et plus délicats que pour le premier par le fait que certaines fonctions du temps qui sont à moyenne nulle sur la durée du cycle cardiaque ne le sont plus sur une portion de cycle et doivent dès lors être prises en compte dans les calculs liés à la méthode des moindres carrés.

On notera qu'il est ainsi possible de déterminer la compliance $\gamma(t)$ sans connaître la pression P(t) qui règne dans l'artère à l'endroit de la mesure. La détermination de P(t) est cependant très souhaitable en soi et aussi en liaison avec les fonctions Q(t) et γ(t).

Un autre but de l'invention est la détermination de la pression instantanée P(t) en un endroit prédéterminé quelconque d'une artère analysée à partir des fonctions instantanées de la compliance γ(t) et du rayon r(t) de l'artère en cet endroit.

A cet effet, un procédé de mesure de la pression : $P(t) = P_0 + p(t)$ à l'intérieur d'une artère suivant lequel la valeur de r(t) pour l'un des tirs A ou B est déterminée à partir des valeurs $r_0$ et $\Delta r(t)$, compatible avec les précédents, est remarquable en ce que la partie variable p(t) de la pression est déterminée par intégration par rapport au temps de la fonction aux dérivées partielles $P_1$ :

$$P_1(t) = \int_t \frac{\partial P}{\partial t} dt = \int_t \frac{1}{\gamma(t)} \frac{\partial r}{\partial t} dt$$

puis suppression de la composante continue $P_c$ de $P_1(t)$ :

$$p(t) = P_1(t) - P_c$$

et que la partie fixe $P_0$ (à ajouter à p(t)) est déterminée par application de la méthode des moindres carrés à la relation :

$$\frac{\partial P}{\partial t} = \frac{1}{r} \frac{\partial T}{\partial t} - \frac{1}{r^2} \frac{\partial r}{\partial t} T$$

dans laquelle : r = r(t) et T = T(t) = r(t) P(t), T(t) étant la tension, au sens physique, exercée par le sang sur une circonférence de l'artère, d'où l'on déduit en première approximation :

$$T_0 = \frac{1}{\gamma_0} \frac{\left\langle r\frac{\partial r}{\partial t}\right\rangle \left\langle \frac{1}{r} \frac{\partial r}{\partial t}\right\rangle - \left\langle (\frac{\partial r}{\partial t})^2\right\rangle}{\left\langle (\frac{1}{r} \frac{\partial r}{\partial t})^2\right\rangle - \left\langle \frac{1}{r} \frac{\partial r}{\partial t}\right\rangle^2},$$

les signes ⟨ ⟩ indiquant la valeur moyenne de la fonction entourée sur un cycle cardiaque, et :

$$P_0 = T_0/r_0 + P_e$$

$P_e$ étant la pression, constante, à l'extérieur de l'artère.

La description qui suit, en regard des dessins annexés, le tout donné à titre d'exemple non limitatif fera bien comprendre comment l'invention peut être réalisée.

La figure 1 est le schéma synoptique d'un mode de réalisation du dispositif selon l'invention.

La figure 2 représente en coupe diamétrale avec arrachement une portion d'artère et deux lignes de tir ultrasonores rapprochées.

Les figures 3 et 4 représentent, sous forme de schémas synoptiques partiels un premier et un deuxième modes de réalisation particuliers du dispositif selon l'invention.

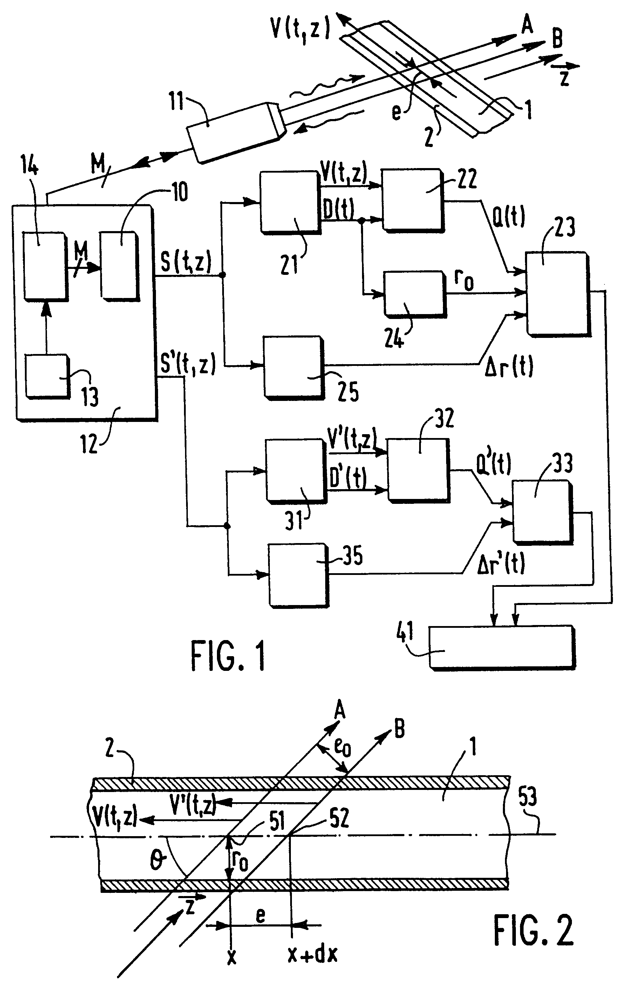

Le schéma de la figure 1 montre un dispositif de mesure de paramètres physiologiques d'un écoulement sanguin 1 dans une artère 2. Ce dispositif comprend un transducteur piézoélectrique 11 qui peut être, par exemple, une barrette multiéléments. Des moyens d'émission-réception 12 reliés au transducteur 11 assurent la formation d'un faisceau ultrasonore d'exploration tandis qu'une première unité de mesure 21 traite les signaux échographiques renvoyés vers le transducteur 11 de façon à fournir une estimée de la vitesse V(t,z) de l'écoulement 1 en fonction du temps t et de la profondeur |$\vec{z}$| d'exploration pour une première ligne de tir A dans la direction $\vec{z}$.

De façon classique, les moyens d'émission-réception comportent au moins un étage d'émission 13 et un séparateur 14 des étages d'émission 13 et de mesure 21. L'étage d'émission 13 comprend un séquenceur composé d'un oscillateur et d'un diviseur de fréquence qui commande, à la fréquence de récurrence $1/T_r$ choisie, un générateur dont les signaux électriques d'excitation sont envoyés vers le transducteur 11, composé de plusieurs (M) transducteurs élémentaires, qui les convertit en trains périodiques de signaux impulsionnels ultrasonores. Le séparateur 14 évite

6

l'aveuglement des circuits de mesure par les signaux d'émission et fournit à un dispositif de formation de voies en réception, 10, les M signaux reçus par les M transducteurs élémentaires. Une commande de balayage électronique (non réprésentée) des transducteurs élémentaires du transducteur 11 permet le choix de telle ou telle ligne de tir, sans déplacement du transducteur 11, ces lignes étant disposées dans un même plan, en éventail ou, comme il est supposé ici, parallèles entre elles. Quant au dispositif de formation de voies il permet, par des configurations de retards particulières imposées aux signaux reçus du séparateur 14, d'obtenir, par focalisations successives, autant de points que prévu sur la ligne de tir choisie en 11, du point le plus proche vers le plus éloigné. Le circuit de formation de voies 10 fournit aux circuits de traitement de signal situés en aval un signal de sortie S(t,z).

L'unité 21 de mesure de la vitesse V(t,z) est décrite en détails de la demande de brevet européen n° 0 458 384 A1 précitée. Elle comporte en entrée, de façon non représentée, un amplificateur et un éliminateur d'échos fixes qui élimine la plus grande partie des échos en provenance des parois de l'artère analysée. Le signal est ensuite traité de préférence selon la méthode dite d'intercorrélation décrite dans la demande de brevet européen n° 0 225 667 précitée, qui utilise de façon connue le principe de la corrélation temporelle, ce qui met en oeuvre un circuit d'intercorrélation et, de préférence, un circuit de multiplexage-interpolation.

Contrairement aux vélocimètres Doppler traditionnels, la vitesse d'écoulement V(t,z) ainsi déterminée présente l'avantage d'être insensible à la dispersion de la fréquence de l'onde ultrasonore utilisée, ce qui permet d'envisager une exploitation beaucoup plus complète des résultats. Les valeurs trouvées pour la vitesse V(t,z) sont, en 21, emmagasinées dans une mémoire de stockage en vue de traitements ultérieurs.

Par ailleurs, le signal de sortie de l'éliminateur d'échos fixes est aussi mis à profit, en 21, pour déterminer le diamètre D(t) de l'écoulement. A partir des fonctions instantanées V(t,z) et D(t), un circuit 22 de mesure du débit instantané Q(t) fournit, à partir des deux signaux de sortie de l'unité 21, l'information de débit sanguin, sous forme d'échantillons temporels numériques, dans des premiers moyens de mémorisation d'échantillons numériques 23.

Le signal D(t) est aussi fourni à l'entrée d'un circuit de moyenne 24 qui fournit à son tour au circuit 23 des échantillons numériques représentatifs du rayon moyen interne de l'artère $r_0(t)$.

A partir du signal S(t,z) il est encore possible de mesurer avec précision un autre paramètre physiologique, qui est la variation de rayon instantanée $\Delta r(t)$, comme décrit dans la demande de brevet européen n° 0 225 667. Pour la mesure de $\Delta r(t)$, il est prévu une deuxième unité de mesure 25 de la vitesse radiale $V_1(t,z)$ et $V_2(t,z)$ de déplacement des deux parois de l'artère qui bornent diamétralement l'écoulement sanguin ainsi que d'autres circuits de mémorisation et de calcul. En 25, de façon non représentée, une première chaîne est constituée par un deuxième amplificateur, une mémoire, puis une chaîne d'intercorrélation semblable à celle indiquée ci-dessus pour la mesure de V(t,z). En l'occurrence il n'y a pas ici d'éliminateur d'échos fixes car c'est précisément les signaux d'écho de paroi que l'on traite à présent, les mouvements des parois étant en substance dans des directions perpendiculaires à celles du sang et le tir A étant effectué obliquement dans un plan méridien de l'artère.

En 25 sont encore calculés d'autres paramètres caractéristiques de l'artère aboutissant au calcul de la fonction variation de rayon instantanée, $\Delta r(t)$. Il s'agit d'abord de l'épaisseur des parois $d_1(t)$ et $d_2(t)$ diamétralement opposées dont la mesure met en oeuvre, en sortie dudit deuxième amplificateur, un circuit de calcul de l'énergie locale et un circuit de calcul des épaisseurs instantanées de parois $d_1$ et $d_2$, constitué par un détecteur de seuil de valeur ajustable. Les vitesses moyennes des parois $\hat{V}_1(t_0)$ et $\hat{V}_2(t_0)$ sont déterminées grâce à des circuits constitués chacun par un additionneur et un diviseur. Enfin, un deuxième intégrateur, (additionneurs) pour intégration dans le temps de la vitesse moyenne, permet d'obtenir avec précision, pour chaque paroi, la fonction déplacement de cette dernière :

$$D_1(t) = \sum_{t_0=0}^{t_0=t} \hat{V}_1(t_0)$$

$$D_2(t) = \sum_{t_0=0}^{t_0=t} \hat{V}_2(t_0)$$

Les vitesses des parois sont très faibles, de l'ordre de 0,5 cm/s.

La fonction recherchée, $\Delta r(t)$, variation de rayon instantané, est alors obtenue, au moyen d'un soustracteur et d'un diviseur par 2, à partir de la relation :

$$\Delta r(t) = 1/2 \, (D_2(t) - D_1(t))$$

relation qui caractérise le déplacement symétrique des parois de l'artère (dilatation ou contraction). Les résultats obtenus sous forme d'échantillons numériques sont eux aussi fournis aux premiers moyens de mémorisation 23.

**EP 0 603 967 B1**

Les éléments 21 à 25 constituent un premier ensemble de mesure de Q(t), $r_0$(t), $\Delta r$(t) à partir des échos ultrasonores engendrés dans l'artère 1 par le tir A.

Selon l'invention, une deuxième ligne de tir B est effectuée, à partir du transducteur 11, dans la même direction $\vec{z}$ et à proximité immédiate de la ligne de tir A. La distance d'écart e, qui sépare les tirs A et B dans la direction de l'axe de l'artère, est typiquement comprise entre quelques dixièmes de mm et quelques mm. Le choix de e est commandé par la longueur de l'onde $\lambda$ de propagation de la systole le long des artères, $\lambda$ étant de l'ordre de 0,5 m. Il faut que e soit très inférieur à $\lambda$, ce qui justifie le choix de distances 100 à 1000 fois plus faibles.

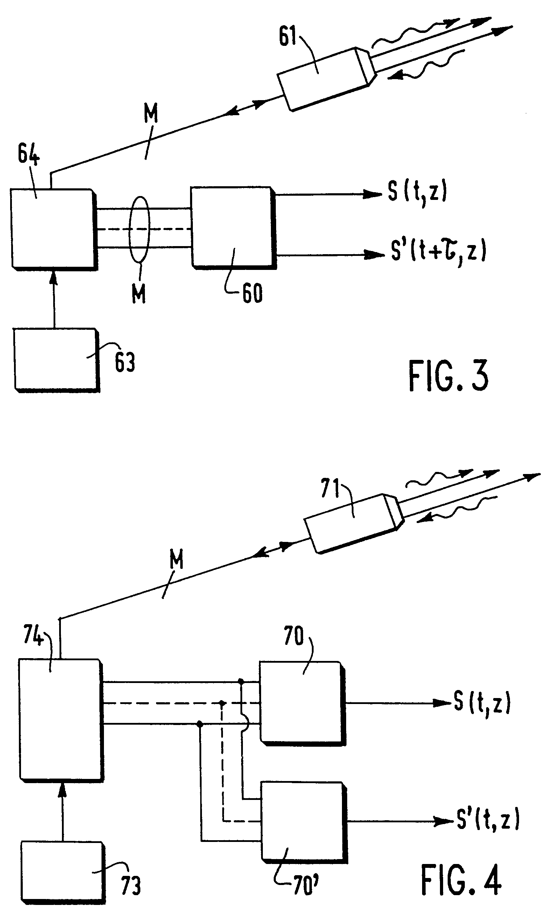

Selon le matériel échographique qu'on met en oeuvre, comme décrit ci-dessous en référence aux figures 3 et 4, les tirs A et B peuvent être soit simultanés, soit légèrement différés d'une durée $\tau$, petite devant la durée $T_c$ d'un cycle cardiaque. Il s'ensuit un deuxième signal de sortie S'(t,z) des moyens d'émission-réception 12, plus précisément du dispositif de formation de voies 10, qui est fourni à un deuxième ensemble de mesure de Q' (t) et $\Delta r'$(t) semblable au premier ensemble de mesure décrit ci-dessus (la mesure de $r_0$, déjà calculée par le premier ensemble, étant ici inutile). Après traitement dans les blocs 31, 32, 35, homologues des blocs 21, 22, 25, le débit sanguin Q'(t) et la variation de tension instantanée $\Delta r'$(t) sont fournis sous forme d'échantillons numériques à des deuxièmes moyens de mémorisation 33.

Des moyens de calcul (calculateur) 41, consistant en un traitement informatique, permettent de déterminer à partir des échantillons numériques contenus en 23 et 33, des paramètres physiologiques caractéristiques de l'artère analysée, notamment l'élasticité $\gamma$(t), définie comme :

$$\gamma\,(t) = \frac{dr(t)}{dP(t)} \qquad\qquad (3)$$

et la pression interne P(t), comme décrit plus loin. A noter que, en cas de tirs A et B non simultanés, un recalage de phase entre échantillons contenus dans les mémoires 23 et 33 est la première opération à effectuer par le calculateur 41, le déphasage à corriger étant égal à : $2\Pi\tau/T_c$ ($T_c$ étant la période du cycle cardiaque).

La figure 2 représente, exagérément grossie pour la clarté du dessin, une partie d'artère dans la région d'analyse échographique selon l'invention. Cette région est délimitée par deux plans radiaux de cote x et x + dx passant par les points 51 et 52 d'intersection des tirs A et B avec l'axe 53 de l'artère, les tirs A et B, parallèles, définissant un plan méridien de l'artère qui est choisi comme plan de coupe de la figure. Ces deux plans sont séparés par la distance $\frac{e_0}{\sin\theta}$, $e_0$ étant la distance entre les deux tirs A et B, et $\theta$ l'angle que fait la direction de l'artère avec la direction $\vec{z}$ des deux tirs. Selon la valeur choisie pour $e_0$ et la valeur de $\theta$, la distance d'écart e est comprise entre quelques dixièmes de mm et quelques mm. On a aussi représenté sur la figure les vitesses ponctuelles du sang V(t,z) et V'(t,z) mesurées dans l'artère dont la lumière, ou section interne, est : $S = \Pi r^2$ (section moyenne : $S_0 = \Pi r_0{}^2$).

Les figures 3 et 4 représentent deux modes de réalisation particuliers du dispositif selon l'invention.

L'échographe de la figure 3 peut être considéré comme connu, pour l'essentiel, dans sa structure, dans la mesure où il se déduit simplement d'un échographe dit CVI (Colour Velocity Imaging). Dans l'application CVI il s'agit de construire, à partir de 128 transducteurs élémentaires, une image échographique, constituée par exemple de 128 lignes parallèles coplanaires, dont les points pour lesquels les vitesses dépassent des seuils prédéterminés positif et négatif sont repérés par des couleurs différentes (cas du sang dans les veines et les artères qui se rapproche ou s'éloigne de la sonde ultrasonore). De préférence, le nombre de lignes à analyser est égal au nombre M de transducteurs élémentaires. Les lignes de l'image sont construites en succession, dans l'ordre de leurs numéros d'ordre. Cependant, afin d'adapter la fréquence de mesure des points successifs de chaque ligne aux plages de vitesses (sanguines) à mesurer, il est optimal de procéder par 2, 3 ou 4 lignes successives de l'image à la fois (après quoi on passe à la construction des 2, 3 ou 4 lignes suivantes). Pour la construction de deux lignes adjacentes (typiquement distantes de 0,45 mm), ce qui nécessite plusieurs tirs, on détermine un point d'une première ligne, puis le point homologue de la deuxième ligne, après un temps $\tau$, puis le point suivant de la première ligne, après un temps $2\tau$, et ainsi de suite jusqu'à épuisement des points de ces deux lignes. Pour ce faire, le dispositif de formation de voies en réception 60, qui reçoit les M signaux d'écho en provenance du transducteur 61 par l'intermédiaire du séparateur 64 commandé par l'étage d'émission 63, reconfigure ses retards, à chaque temps $\tau$, tantôt sur la première ligne, tantôt sur la suivante. Les signaux obtenus pour les deux lignes précitées suffisent pour la mise en oeuvre de la présente invention. Ces signaux, dont le démultiplexage est effectué de façon connue en 60, sont notés S(t,z) et S'(t+$\tau$,z) en sortie du dispositif de formation de voies 60. En toute rigueur, les signaux de sortie doivent être simultanés pour les points homologues de la paire de lignes analysée. En conséquence, une remise en phase égale à : $2\Pi\tau/T_c$ est effectuée, après traitement des signaux, entre les échantillons numériques contenus dans la mémoire 33 (figure 1) et ceux contenus dans la mémoire 23.

Toujours en utilisant de façon sélective pour les lignes le mode de fonctionnement CVI il est aussi possible de combiner deux lignes voisines séparées par 2 fois le pas des lignes (0,9 mm), le déphasage à corriger étant égal à

: $4\Pi\tau/T_c$, ou 3 fois ce pas (1,35 mm), avec un déphasage à corriger de $6\Pi\tau/T_c$. Aussi, pour la caractérisation d'un athérome ou d'une sténose, on peut prévoir d'analyser, conformément à la présente invention, sans déplacement de la sonde, plusieurs paires de lignes successives, de façon à déterminer la compliance $\gamma$ et la pression P en plusieurs points rapprochés de l'artère, sur une distance axiale de l'ordre de 1 à 3 cm.

Le mode de réalisation de la figure 4 est d'un fonctionnement plus simple que celui de la figure 3. Il s'agit pour l'essentiel d'un profilomètre, conçu à l'origine pour effectuer une seule ligne de tir, au moyen du transducteur 71 à M tranducteurs élémentaires, de l'étage d'émission 73, du séparateur 74, et du dispositif de formation de voies 70 qui fournit le signal S(t,z). Ce profilomètre est adapté pour fournir, en réception, une deuxième ligne d'analyse, voisine et parallèle à la première, S'(t,z,). A cette fin, il est en premier lieu nécessaire d'émettre un faisceau un peu plus large, c'est-à-dire légèrement défocalisé et un peu plus puissant que pour un profilomètre conventionnel. La légère défocalisation est peu pénalisante en ce qui concerne la précision requise pour les mesures des points analysés. Cette défocalisation s'obtient simplement par réglage approprié de la commande de balayage électronique (non représentée) des transducteurs élémentaires, lors des tirs en impulsions, en 71. Moyennant l'émission adaptée que l'on vient de décrire, un deuxième dispositif de formation de voies 70', qui reçoit les mêmes signaux que le dispositif 70 permet, avec une configuration de retards légèrement décalée par rapport à celle du dispositif 70, de construire en réception une deuxième ligne d'analyse voisine, parallèle à la première, et que l'on peut identifier par le signal de sortie S'(t,z). Dans ce mode de réalisation, la deuxième ligne est obtenue simultanément à la première, en ce sens que chaque paire de points homologues des deux lignes est fournie au même instant en sortie des dispositifs 70 et 70'. On peut prévoir de rendre réglable le décalage temporel entre les configurations de retards appliquées dans les dispositifs de formation de voies 70 et 70', ce qui se traduit par une variation de la distance d'écart e, laquelle se déduit directement de ce décalage temporel. Le doublement des dispositifs de formation de voies rend le dispositif de la figure 4 plus cher que celui de la figure 3 même si, dans ce cas, le recalage temporel des points homologues de deux lignes n'est plus nécessaire.

L'invention a pour but de mesurer l'élasticité $\gamma$ d'une artère sous l'effet de la pression sanguine P, à partir des échantillons numériques contenus dans les mémoires 23 et 33 (figure 1).

L'équation fondamentale de la dynamique, transposée à la mécanique circulatoire, peut s'écrire, en ce qui concerne le flux sanguin :

$$F_R + dx\ \rho\ \frac{dV}{dt}\ dS = [-\ P(x+dx) + P(x)]dS$$

avec :

$$
\begin{array}{ll}
\rho & : \text{masse volumique du sang} \\
V = V(t,x) & : \text{vitesse du sang} \\
P = P(t,x) & : \text{pression sanguine} \\
F_R & : \text{forces de frottements}
\end{array}
$$

En intégrant par rapport à S et après division par S et par dx :

$$F'_R + \frac{\rho}{S}\int_S \frac{dV}{dt}\ dS = -\ \frac{dP}{dx} \tag{5}$$

par ailleurs :

$$\frac{dV}{dt} = \frac{\partial V}{\partial t} + V\ \frac{\partial V}{\partial x}$$

la relation (5) peut s'écrire :

$$RQ + L\left[\frac{\partial Q}{\partial t} + \frac{\partial U}{\partial x}\right] = -\ \frac{dp}{dx} \tag{6}$$

avec :

$Q = Q(t,x)$ : débit sanguin

R : résistance hydrodynamique mettant en jeu les frottements sur la paroi artérielle et la viscosité du sang,

$$L = \rho/S$$

$$\sqcup = \frac{1}{2} \int V^2 dS$$

Avec une très bonne approximation, on supposera pour la suite des calculs que la fonction $\sqcup(x)$ est une constante dans le cas du sang, soit :

$$\frac{\partial \sqcup}{\partial x} \approx 0$$

et la relation (6) simplifiée devient :

$$RQ + L \frac{\partial Q}{\partial t} = - \frac{dP}{dx} \qquad (7)$$

On notera qu'avec cette simplification une seule fonction parmi Q(t) ou Q'(t) suffit pour la suite des calculs.
Par ailleurs la pression P agit aussi sur les parois artérielles en les dilatant ou en les comprimant, mettant en jeu leur élasticité ou compliance γ, paramètre essentiel décrivant les performances du système artériel. Concernant les parois de l'artère, on peut écrire :

$$\frac{dP}{dx} = \frac{\partial P}{\partial x} + \frac{\partial P}{\partial S} \frac{\partial S}{\partial x} = \frac{\partial P}{\partial x} + \frac{\partial P}{\partial r} \frac{\partial r}{\partial x}$$

soit, avec :

$$\gamma = \frac{\partial r}{\partial P}$$

et en posant

$$\frac{\partial P}{\partial x} = \frac{\Delta P}{\Delta x}$$

pour la relation (7) :

$$\gamma \left[ RQ + L \frac{\partial Q}{\partial t} + \frac{\Delta P}{\Delta x} \right] = - \frac{\partial r}{\partial x} \qquad (8)$$

L'invention repose sur la possibilité physique de mesurer les quantités r(x) et r(x + dx) avec une précision suffisante pour que leur différence soit bien significative. En pratique la précision sur cette différence instantanée peut être obtenue à partir de l'expression :

$$\delta r(t) = \Delta r(t) - \Delta r'(t)$$

et, dans la relation (8) on fait l'identification :

$$\frac{\partial r}{\partial x} = \frac{\delta r(t)}{e} \qquad (9)$$

On notera que la quantité dx/dt = e/dt est la vitesse de propagation de l'onde systolique le long de l'artère (de l'ordre de 6 m/s) et non la vitesse du sang à l'intérieur de cette dernière, soit, pour une valeur e de 0,45 mm, la valeur : dt = 75 µs, ce qui justifie l'existence d'une valeur δr mesurable avec une valeur de dx aussi faible.

Dans la relation (8) sont d'abord calculées les quantités $\partial Q/\partial t$ (à partir de la fonction connue Q(t)) et $-\partial r/\partial x$ (selon l'égalité (9)).

On se propose maintenant d'estimer, dans un premier temps les valeurs moyennes des paramètres $\gamma$, R et $\Delta P/\Delta x$, notées $\gamma_0$, $R_0$ et $(\frac{\Delta P}{\Delta x})_0$.
$\Delta P/\Delta x$ représente la composante continue de la fonction $\frac{dP(x,t)}{dx}$, c'est-à-dire le gradient de pression moyen le long de l'artère, engendré par le débit moyen : $Q_0 = \langle Q(t)\rangle$, calculable à partir de la fonction Q(t), soit : $R_0Q_0$. Par ailleurs, la variation de r étant faible, sur la distance e, on peut poser :

$$L = L_0 = \frac{\rho}{\Pi r_0^2} \quad \text{(en g, mm).}$$

Cette estimation est effectuée de préférence par la méthode des moindres carrés, ce qui revient à rendre minimal le carré de la norme :

$$\left\| T_1(Q) + \frac{\partial r}{\partial x}\right\|^2$$

où :

$$T_1(Q) = \gamma\left[R Q + L0\frac{\partial Q}{\partial t} + R Q_0\right]$$

il en découle trois équations aux dérivées partielles :

$$\frac{\partial}{\partial var}\left(\left\|T_1(Q) + \frac{\partial r}{\partial x}\right\|^2\right) = 2\left\langle \frac{\partial T_1(Q)}{\partial var} \cdot \left(T_1(Q) + \frac{\partial r}{\partial x}\right)\right\rangle = 0$$

avec, successivement, var = $\gamma$, R et $RQ_0$.
Tous calculs faits, on en déduit (les valeurs moyennes étant prises sur la durée $T_c$ d'un cycle cardiaque) :

$$\gamma_0 = \frac{1}{L_0}\frac{\left\langle\frac{\partial Q}{\partial t}\times\frac{\partial r}{\partial x}\right\rangle}{\left\langle\frac{\partial Q^2}{\partial t}\right\rangle} = \frac{b}{L_0} \quad \text{(en g, mm, s)}$$

la compliance $\gamma$ s'exprime de préférence en microns par millimètres de hauteur de mercure (µ/mm Hg) ce qui entraîne, avec $\rho = 10^{-3}$ g/mm$^3$ :

$$\gamma_0 = 419,14\times 10^3 r_0^2 b \text{ µ/mm Hg } (r_0, b \text{ en mm, s})$$

les valeurs trouvées pour $\gamma_0$ sont de l'ordre de 1 µ pour 40 µ de mercure. Par ailleurs la méthode des moindres carrés précitée fournit la relation :

$$a = \gamma_0 \, R_0 = - \frac{\left\langle Q \times \dfrac{\partial r}{\partial x} \right\rangle}{\left\langle Q^2 \right\rangle}$$

Il s'ensuit que :

$$R_0 = \frac{a}{\gamma_0} = L_0 \, \frac{a}{b} = - L_0 \, \frac{\left\langle Q \times \dfrac{\partial r}{\partial x} \right\rangle \times \left\langle \dfrac{\partial Q^2}{\partial t} \right\rangle}{\left\langle Q^2 \right\rangle \times \left\langle \dfrac{\partial Q}{\partial t} \times \dfrac{\partial r}{\partial x} \right\rangle}$$

et :

$$\left( \frac{\Delta P}{\Delta x} \right)_0 = R_0 Q_0 = \frac{a}{\gamma} \, Q_0$$

ou encore :

$$\left( \frac{\Delta P}{\Delta x} \right)_0 = \frac{1}{419,14} \times \frac{Q_0}{r_0^2} \times \frac{a}{b} \quad \text{mm Hg/mm}$$

Pour une artère saine, le gradient de pression $R_0 Q_0$ est sensiblement constant tout le long de l'artère, c'est-à-dire depuis le coeur jusqu' aux organes. Par contre, à l'endroit d'une sténose, le gradient de pression va être nettement plus élevé qu'en d'autres points de l'artère. La détermination de ce gradient telle qu'indiquée ci-dessus permet au praticien de localiser avec précision une sténose et de pouvoir la répertorier, en répétant plusieurs fois les mesures précitées, à intervalles de quelques millimètres, le long de la partie altérée de l'artère, ce qui permet d'obtenir un profil du gradient de pression. Il devient ainsi possible d'envisager, par un procédé non invasif quels types de soins ou d'intervention (angioplastie) conviennent pour le traitement de cette sténose. On peut aussi détecter ainsi avec précision un anévrisme.

Si la valeur moyenne du gradient de pression constitue déjà une indication très précise, on peut cependant souhaiter connaître la variation en fonction du temps des fonctions R(t) et γ(t) au cours du cycle cardiaque. Ceci peut être obtenu, à partir des valeurs moyennes préalablement déterminées : $L_0$, $\gamma_0$, $R_0$ et $R_0 Q_0$ en appliquant à nouveau la méthode des moindres carrés, non plus sur toute la durée d'un cycle cardiaque mais pour deux demi-cycles puis, par itérations successives pour quatre, huit, ..., parties de cycles. Pour ce faire, on part de la relation (8) dans laquelle on pose :

$$R = R_0 (1 + R'(t))$$

$$\gamma = \gamma_0 (1 + g'(t))$$

$$\frac{\partial r}{\partial x} = \frac{\delta r(t)}{e}$$

soit :

$$\gamma_0 (1 + g'(t)) \left[ R_0 (1 + R'(t)) Q(t) + L_0 \frac{\partial Q}{\partial t} + R_0 \, Q_0 \right] = - \frac{\delta r(t)}{e}$$

Ce procédé permet de fournir pour $\gamma$ et R des fonctions constantes par morceaux, c'est-à-dire, sous forme de quelques échantillons sur la durée du cycle cardiaque, $\gamma(t)$ et $R(t)$.

On notera que les calculs sont ici rendus plus complexes par le fait que certaines fonctions qui étaient à moyenne nulle sur la durée du cycle cardiaque pour le calcul de $\gamma_0$ et $R_0$, ne le sont plus pour une partie du cycle.

Connaissant la compliance en fonction du temps, par sa fonction approchée $\gamma(t)$, la pression interne dans l'artère en fonction du temps $P(t)$ peut être calculée, par intégration A partir de la définition même de la compliance $\gamma$ :

$$\gamma = \frac{\partial r}{\partial P} \qquad (3)$$

soit :

$$\frac{\partial P}{\partial t} = \frac{1}{\gamma} \frac{\partial r}{\partial t} \qquad (2)$$

et :

$$P_1(t) = \int_t \frac{\partial P}{\partial t} dt = \int_t \frac{1}{\gamma(t)} \frac{\partial r}{\partial t} dt \qquad (11)$$

La notation $P_1(t)$ exprime le fait que la pression recherchée $P(t)$ est obtenue, suite à l'intégration, à une constante additive près, ou composante continue $P_c$ :

$$P_c = \langle P_1(t) \rangle$$

(valeur moyenne calculée sur un cycle cardiaque). Si l'on pose :

$$P(t) = P_0 + p(t)$$

la partie variable $p(t)$ de la pression $P(t)$ s'obtient par différence :

$$p(t) = P_1(t) - \langle P_1(t) \rangle \qquad (12)$$

La valeur moyenne ou partie fixe $P_0$ s'obtient quant à elle en faisant intervenir la Tension $T(t)$, au sens physique (et non médical) du terme, exercée par le sang sur une circonférence de l'artère :

$$P(t) - P_e = T(t)/r(t) \text{ (pression transmurale)} \qquad (13)$$

avec $P_e$ : pression externe à l'artère, constante, de l'ordre de la pression atmosphérique.

Par dérivation par rapport au temps :

$$\frac{\partial P}{\partial t} = \frac{1}{r} \frac{\partial T}{\partial t} - \frac{1}{r^2} \frac{\partial r}{\partial t} T \qquad (14)$$

relation dans laquelle : $P = P(t)$, $T = T(t)$ et $r = r(t)$. Soit, en multipliant par r et en prenant en compte l'équation (2) :

$$\frac{\partial T}{\partial t} - \frac{1}{r} \frac{\partial r}{\partial t} T = \frac{r}{\gamma} \frac{\partial r}{\partial t}$$

La méthode des moindres carrés est à nouveau utilisée. Soit à rendre minimal le carré de la norme :

$$\left\| T_2(T) - \frac{r}{\gamma} \frac{\partial r}{\partial t} \right\|^2$$

où :

$$T_2(T) = \frac{\partial T}{\partial t} - \frac{1}{r}\frac{\partial r}{\partial t} T$$

Il en découle deux équations aux dérivées partielles :

$$\frac{\partial}{\partial var}\left(\left\|T_2(T) - \frac{r}{\gamma}\frac{\partial r}{\partial t}\right\|^2\right) = 2 \left\langle \frac{\partial T_2(T)}{\partial var} \cdot \left(T_2(T) - \frac{r}{\gamma}\frac{\partial r}{\partial t}\right)\right\rangle = 0$$

avec, successivement : var = $\partial T/\partial t$ et var = T

Tous calculs faits on en déduit en faisant, à ce stade, la simplification : $\gamma = \gamma_0$, la valeur moyenne $T_0$ de T :

$$T_0 = \frac{1}{\gamma_0} \frac{\left\langle r\frac{\partial r}{\partial t}\right\rangle \left\langle \frac{1}{r}\frac{\partial r}{\partial t}\right\rangle - \left\langle (\frac{\partial r}{\partial t})^2\right\rangle}{\left\langle (\frac{1}{r}\frac{\partial r}{\partial t})^2\right\rangle - \left\langle \frac{1}{r}\frac{\partial r}{\partial t}\right\rangle^2} \qquad (15)$$

Il s'ensuit que :

$$P_0 = P_e + T_0/r_0$$

d'où l'on déduit finalement :

$$P(t) = P_1(t) - \langle P_1(t) \rangle + P_e + T_0/r_0$$

Selon la fonction choisie pour le rayon, r(t) (cote x) ou r'(t) (cote x+dx), est ainsi obtenue la pression P(t) au point 51 (figure 2) ou au point 52. On notera par ailleurs que les fonctions $\partial r/\partial t$ peuvent être obtenues par dérivation directe des fonctions $\Delta r(t)$ ou $\Delta r'(t)$, en ce qui concerne les calculs de moyennes à effectuer selon la relation (15).

## Revendications

1. Echographe ultrasonore utilisé en profilomètre (mode M) et muni de moyens d'émission-réception (12) incorporant un dispositif de formation de voies en réception (10), muni d'un dispositif de mesure de paramètres physiologiques d'une artère sous l'effet de la pression sanguine P comportant un premier ensemble de mesure et de calcul du débit sanguin instantané Q(t) (22), de la variation de rayon instantanée $\Delta r(t)$ de ladite artère (25) et de son rayon moyen $r_0$ (24) selon une première ligne de tir A, à partir de mesures de profondeurs d'exploration z pour le sang ou les parois de l'artère (2) obtenues avec une précision de l'ordre du micron, caractérisé en ce que ledit dispositif de mesure comporte en outre, pour la détermination de l'élasticité instantanée $\gamma$ (t) de ladite artère et de la pression instantanée P(t) à l'intérieur de cette dernière, par une méthode mettant en oeuvre l'équation fondamentale de la dynamique transposée à la mécanique circulatoire, un deuxième ensemble de mesures et de calcul par échographie ultrasonore des paramètres instantanés Q'(t) (32) et $\Delta r'(t)$ (35), simultanés ou recalés dans le temps par rapport aux valeurs instantanées Q(t) et $\Delta r(t)$, selon une deuxième ligne de tir B située à une distance d'écart e de la première ligne de tir A, comprise entre 2 dixièmes de millimètres et 9 millimètres, assez petite pour constituer une mesure différentielle, les deux lignes de tir A et B traversant l'artère suivant un de ses plans méridiens, la distance d'écart e étant prise dans la direction de l'axe (53) de l'artère en vue du calcul de la valeur $\frac{\Delta r(t) - \Delta r'(t)}{e}$, des premiers (23) et deuxièmes (33) moyens de mémorisation pour le stockage d'échantillons numériques de signaux relatifs aux flux sanguins et de signaux relatifs aux parois de l'artère étant prévus en sortie desdits premier et deuxième ensembles de mesure, ainsi que des moyens (41) de calcul effectués à partir desdits échantillons numériques de signaux.

2. Echographe ultrasonore muni d'un dispositif de mesure de paramètres physiologiques d'une artère selon la revendication 1, caractérisé en ce qu'il comporte des moyens d'émission-réception (12) fonctionnant selon le principe de la corrélation temporelle et destiné à la fourniture d'images ultrasonores en mode bidimensionnel, dont la détection, limitée à deux lignes de tir voisines A et B séparées par ladite distance d'écart e, est effectuée paire par paire de points homologues des deux lignes de tir, ainsi que des moyens (41) de recalage de phase des signaux de réception desdites deux lignes de tir voisines intégrés dans lesdits moyens de calcul (41).

3. Echographe ultrasonore muni d'un dispositif de mesure de paramètres physiologiques d'une artère selon la revendication 1, caractérisé en ce qu'il fonctionne selon le principe de la corrélation temporelle, et qu'il est utilisé en profilomètre dans lequel les moyens d'émission (73, 74) sont adaptés pour émettre un faisceau ultrasonore plus puissant et légèrement défocalisé par rapport à ceux d'un profilomètre conventionnel à un seul tir à la fois, et qui incorpore un dispositif de formation de voies en réception doublé (70, 70') pour fournir en parallèle et simultanément en sortie des deux dispositifs de formation de voies les signaux de deux lignes de tir A et B séparées par la distance d'écart e.

4. Procédé de mesure de l'élasticité γ d'une artère sous l'effet de la pression sanguine P, au moyen d'un échographe ultrasonore utilisé en profilomètre (mode M) et muni de moyens d'émission-réception incorporant un dispositif de formation de voies en réception et apte à fournir le débit sanguin instantané Q(t), la variation de rayon instantanée $\Delta r(t)$ et le rayon moyen $r_o$ pour deux lignes de tir parallèles rapprochées A et B traversant l'artère (2) suivant un de ses plans méridiens, pour la mise en oeuvre de l'échographe ultrasonore muni du dispositif de mesure selon l'une des revendications 1 à 3, lesdites valeurs Q(t), $\Delta r(t)$ et $r_o$ étant stockées dans lesdits premiers (23) et deuxième (33) moyens de mémorisation, comportant les étapes suivantes :

i - le calcul, à partir de l'une des deux fonctions Q(t), Q'(t), de la valeur moyenne $Q_0$ et de la dérivée : $\frac{\partial Q(t)}{\partial t}$ de cette fonction.

ii - le calcul, à partir des valeurs instantanées $\Delta r(t)$, $\Delta r'(t)$ pour les lignes A et B de la valeur instantanée :

$$\delta r(t) = \Delta r(t) - \Delta r'(t)$$

iii - la détermination, à partir de la méthode des moindres carrés appliquée, sur un cycle cardiaque, à la relation :

$$\gamma \left[ R\,Q(t) + L\frac{\partial Q(t)}{\partial t} + \frac{\Delta P}{\Delta x} \right] = -\frac{\partial r(t)}{\partial x}$$

dans laquelle est effectuée l'identification :

$$L = \frac{\rho}{\Pi\, r_0^2}, \text{ et } \frac{\partial\, r(t)}{\partial\, x} = \frac{\delta\, r(t)}{e}$$

des valeurs moyennes des paramètres d'élasticité γ, de résistance hydrodynamique R et de gradient de pression $\Delta P/\Delta x$, soit $\gamma_0$, $R_0$ et $R_0 Q_0$ respectivement.

5. Procédé de mesure de l'élasticité γ d'une artère selon la revendication 4, caractérisé en ce que, pour la détermination par morceaux sur au moins un cycle cardiaque des fonctions γ(t) et R(t), la méthode des moindres carrés est à nouveau appliquée par itérations successives sur des parties complémentaires du cycle cardiaque en nombre croissant, d'une itération à la suivante, en prenant, pour la première itération, deux demi cycles cardiaques (puis 4, 8, ..., parties de cycles pour les itérations suivantes), et la relation :

$$\gamma_0(1 + g'(t)) \left[ R_0(1 + R'(t))Q(t) + L_0\frac{\partial Q}{\partial t} + R_0 Q_0 \right] = -\frac{\delta r(t)}{e},$$

ce qui fournit pour γ et R des fonctions échantillonnées de quelques échantillons sur la durée du cycle cardiaque.

6. Procédé de mesure de la pression : P(t) = $P_0$ + p(t) à l'intérieur d'une artère (2) suivant lequel la valeur de r(t) pour l'un des tirs A ou B est déterminée à partir des valeurs $r_0$ et $\Delta r(t)$, selon la revendication 5, caractérisé en ce que la partie variable p(t) de la pression est déterminée par intégration par rapport au temps de la fonction aux dérivées partielles $P_1$ :

$$P_1(t)=\int_t \frac{\partial P}{\partial t}dt=\int_t \frac{1}{\gamma(t)}\frac{\partial r}{\partial t}dt$$

puis suppression de la composante continue $P_c$ de $P_1(t)$ :

$$p(t) = P_1(t) - P_c$$

et que la partie fixe $P_0$ (à ajouter à p(t)) est déterminée par application de la méthode des moindres carrés à la relation :

$$\frac{\partial P}{\partial t}=\frac{1}{r}\frac{\partial T}{\partial t}-\frac{1}{r^2}\frac{\partial r}{\partial t}T$$

dans laquelle : r = r(t) et T = T(t) = r(t) P(t),
T(t) étant la tension, au sens physique, exercée par le sang sur une circonférence de l'artère, d'où l'on déduit en première approximation :

$$T_o = \frac{1}{\gamma_o}\frac{\left\langle r\frac{\partial r}{\partial t}\right\rangle\left\langle \frac{1}{r}\frac{\partial r}{\partial t}\right\rangle-\left\langle \left(\frac{\partial r}{\partial t}\right)^2\right\rangle}{\left\langle \left(\frac{1}{r}\frac{\partial r}{\partial t}\right)^2\right\rangle-\left\langle \frac{1}{r}\frac{\partial r}{\partial t}\right\rangle^2}$$

les signes $\langle\ \rangle$ indiquant la valeur moyenne de la fonction entourée sur un cycle cardiaque, et :

$$P_o = T_o/r_o + P_e$$

$P_e$ étant la pression, constante, à l'extérieur de l'artère.

**Claims**

1. An ultrasonic echograph used in the profilometer mode (M mode) and provided with transmission/receiving means (12), including a device for forming channels in the receiving mode (10), provided with a device for measuring physiological parameters of an artery under the effect of the blood pressure P which comprises a first assembly for measurement and calculation of the instantaneous blood flow rate Q(t) (22), of the instantaneous variation of the radius Δr(t) of said artery (25), and of its mean radius $r_o$ (24) along a first excitation line A, on the basis of measurements of the scanning depths z for the blood or the walls of the artery (2), obtained with a precision of the order of a micron, characterized in that said measuring device also comprises, for the determination of the instantaneous elasticity γ(t) of said artery and the instantaneous pressure P(t) within the latter, by means of a method utilizing the basic dynamics equation transposed to the circulatory system, a second assembly for measurement and calculation by ultrasonic echography of the instantaneous parameters Q'(t) (32) and Δr'(t) (35), simultaneous or locked in time relative to the instantaneous values Q(t) and Δr (t), along a second excitation line B which is situated at a distance e from the first excitation line A, which distance is between 2 tenths of a millimeter and 9 millimeters, being small enough so as to constitute a differential measurement, the two excitation lines A and B traversing the artery according to one of its meridian planes, the distance e being taken in the direction of the axis (53) of the artery with a view to the calculation of the value $\frac{\Delta r(t-\Delta r'(t)}{e}$, first (23) and second (33) storage means for storing digital samples of signals relating to the blood flows and signals relating to the walls of the artery being connected to the output of said first and second measuring assemblies, as well as calculation means (41) which operate on said digital signal samples.

2. An ultrasonic echograph provided with a device for measuring physiological parameters of an artery as claimed in Claim 1, characterized in that it comprises transmission/receiving means (12) operating according to the time correlation principle and intended to produce ultrasonic images in the two-dimensional mode, its detection being limited to two neighbouring excitation lines A and B which are separated by said distance e and taking place in pairs of homologous points of the two excitation lines, as well as means (41) for rephasing the signals received for said two neighbouring excitation lines, which means are integrated in said calculation means (41).

**3.** An ultrasonic echograph provided with a device for measuring physiological parameters of an artery as claimed in Claim 1, characterized in that it operates according to the time correlation principle and is used as a profilometer in which the transmission means (73,74) are arranged to transmit a more powerful and slightly defocused ultrasonic beam in comparison with that of a conventional profilometer with only a single excitation at z time, the device incorporating a device for forming double receiving channels (70,70') to supply, in parallel and simultaneously at the output of the two channel forming devices, the signals of two excitation lines A and B which are separated by the distance e.

**4.** A method for measuring the elasticity $\gamma$ of an artery under the effect of the blood pressure P by means of an ultrasonic echograph used as a profilometer (M mode) and provided with transmission/receiving means comprising a device for forming channels in the receiving mode and arranged to produce the instantaneous blood flow rate Q(t), the instantaneous radius variation $\Delta r$ (t) and the mean radius $r_0$ for two neighbouring, parallel excitation lines A and B traversing the artery (2) according to one of its meridian planes, in order to be carried out by the ultrasonic echograph provided with the measuring device claimed in one of the Claims 1 to 3, said values Q(t), $\Delta r$ (t) and $r_0$ being stored in said first (23) and second (33) storage means, comprising the following steps:

i- the calculation, on the basis of one of the two functions Q(t), Q'(t), of the mean value $Q_0$ and the derivative $\frac{\partial Q(t)}{\partial t}$ of this function,

ii- the calculation, on the basis of the instantaneous values $\Delta r$ (t), $\Delta r'$ (t) for the lines A and B, of the instantaneous value:

$$\delta r(t) = \Delta r(t) - \Delta r'(t)$$

iii- the determination of the mean values of the elasticity parameter $\gamma$, the hydrodynamic resistance parameter R and the pressure gradient $\Delta P/\Delta x$, *i.e.* $\gamma_0$, $R_0$ and $R_0 Q_0$, respectively, on the basis of the least squares method applied, over a cardiac cycle, to the relation:

$$\gamma\left[ RQ(t) + L\frac{\partial Q(t)}{\partial t} + \frac{\Delta P}{\Delta x}\right] = -\frac{\partial r(t)}{\partial x}$$

in which the identification:

$$L = \frac{\rho}{\prod r_0^2}, and \ \frac{\partial r(t)}{\partial x} = \frac{\delta r(t)}{e}$$

is made.

**5.** A method of measuring the elasticity $\gamma$ of an artery as claimed in Claim 4, characterized in that for the determination in segments, over at least one cardiac cycle, of the functions $\gamma(t)$ and R(t) the least-squares method is applied again by successive iterations over the complementary parts of the cardiac cycle whose number increases from one iteration to the next by taking, for the first iteration, two cardiac half-cycles (and then 4, 8, ..., parts of cycles for the subsequent iterations), and the relation:

$$\gamma_0(1 + g'(t))\left[ R_0(1 + R'(t))Q(t) + L_0\frac{\partial Q}{\partial t} + R_0 Q_0 \right] = -\frac{\delta r(t)}{e},$$

which supplies for $\gamma$ and R the sampled functions of some samples over the duration of the cardiac cycle.

**6.** A method for measuring the pressure $P(t) = P_0 + P(t)$ within an artery (2), in which the value of r (t) for one of the excitations A or B is determined on the basis of the values $r_0$ and $\Delta r$ (t) as claimed in Claim 5, characterized in that the variable part p(t) of the pressure is determined by integration with respect to time of the function with the partial derivatives $P_1$:

$$P_1(t) = \int_t \frac{\partial P}{\partial t} dt = \int_t \frac{1}{\gamma(t)} \frac{\partial r}{\partial t} dt$$

followed by suppression of the continuous component $P_c$ of $P_1(t)$:

$$p(t) = P_1(t) - P_c$$

and that the fixed part $P_0$ (to be added to p(t)) is determined by application of the least-squares method to the relation:

$$\frac{\partial P}{\partial t} = \frac{1}{r} \frac{\partial T}{\partial t} - \frac{1}{r^2} \frac{\partial r}{\partial t} T$$

in which: r = r (t) and T = T(t) = r (t) P(t),
T(t) being the tension, in a physical sense, exerted by the blood on a circumference of the artery, so that therefrom in a first approximation there is deduced:

$$T_0 = \frac{1}{\gamma_0} \frac{\langle r \frac{\partial r}{\partial t} \rangle \langle \frac{1}{r} \frac{\partial r}{\partial t} \rangle - \langle \left( \frac{\partial r}{\partial t} \right)^2 \rangle}{\langle \left( \frac{1}{r} \frac{\partial r}{\partial t} \right)^2 \rangle - \langle \frac{1}{r} \frac{\partial r}{\partial t} \rangle^2} \ ,$$

in which the symbols $\langle \ \rangle$ indicate the mean value of the function taken over a cardiac cycle, and:

$$P_0 = T_0 / r_0 + P_e$$

$P_e$ being the (constant) pressure outside the artery.

## Patentansprüche

1. Als Oberflächenmessgerät (M-Mode) genutzter Ultraschall-Echograph, ausgestattet mit Sende-/Empfangsmitteln (12) mit einer Vorrichtung zur Bildung von Wegen im Empfangsmodus (10), der folgendes enthält: eine Vorrichtung zur Messung der physiologischen Parameter einer Arterie unter der Wirkung des Blutdrucks P mit einer ersten Anordnung zur Messung und Berechnung der momentanen Blutdurchflussmenge Q(t) (22), der momentanen Schwankung des Radius $\Delta r(t)$ der genannten Arterie (25) und ihres mittleren Radius $r_0$ (24) entsprechend einer ersten Anlotlinie A ausgehend von den Werten der Eindringtiefe z für das Blut oder die Arterienwände (2), die man mit einer Genauigkeit in der Größenordnung von Mikron erhält, dadurch gekennzeichnet, dass die genannte Messvorrichtung außerdem zur Bestimmung der momentanen Elastizität $\gamma(t)$ der genannten Arterie und des momentanen Drucks P(t) im Inneren der Arterie durch ein Verfahren, bei dem die Fundamentalgleichung der Dynamik auf die Biomechanik des Kreislaufs angewendet wird, eine zweite Anordnung zur Messung und Berechnung der momentanen Parameter Q'(t) (32) und $\Delta r'(t)$ (35) durch Ultraschall-Echographie umfasst, die zeitgleich oder zeitlich versetzt in Bezug auf die momentanen Werte Q(t) und $\Delta r(t)$ sind, und zwar auf einer zweiten Anlotlinie B, die sich in einem Abstand e - der zwischen 2 zehntel Millimetern und 9 Millimetern liegt und klein genug ist, um ein differentielles Maß darzustellen - von der ersten Anlotlinie A entfernt befindet, wobei die beiden Anlotlinien A und B die Arterie in einer ihrer Meridianebenen durchqueren und der Abstand e in der Richtung der Arterienachse (53) gemessen wird, um den Wert $\frac{\Delta r(t) - \Delta r'(t)}{e}$ zu berechnen, wobei erste (23) und zweite (33) Mittel zur Speicherung der digitalen Abtastwerte der Signale in Bezug auf den Blutfluss und der Signale in Bezug auf die Arterienwand am Ausgang der ersten und der zweiten Messanordnung vorgesehen sind, ebenso wie Mittel (41) zur Berechnung ausgehend von den genannten digitalen Abtastwerten der Signale.

2. Ultraschall-Echograph mit einer Vorrichtung zur Messung der physiologischen Parameter einer Arterie nach Anspruch 1, <u>dadurch gekennzeichnet</u>, dass er Mittel zum Aussenden/Empfangen (12) enthält, die nach dem Prinzip der zeitlichen Korrelation funktionieren und vorgesehen sind, um im zweidimensionalen Betrieb Ultraschallbilder zu liefern, die - begrenzt auf zwei benachbarte Anlotlinien A und B, die durch einen Abstand e voneinander getrennt sind - durch paarweise Ermittlung der homologen Punkte der beiden Anlotlinien detektiert werden, und Mittel (41), die vorgesehen sind, um die Empfangssignale der genannten beiden benachbarten Anlotlinien wieder in Phase zu bringen, und in die genannten Berechnungsmittel (41) integriert sind.

3. Ultraschall-Echograph mit einer Vorrichtung zur Messung der physiologischen Parameter einer Arterie nach Anspruch 1, <u>dadurch gekennzeichnet</u>, dass er nach dem Prinzip der zeitlichen Korrelation arbeitet und als Oberflächenmessgerät benutzt wird, in dem die Aussendungsmittel (73, 74) angepasst sind, um einen Ultraschallstrahl auszusenden, der stärker und im Vergleich zum Strahlenbündel eines herkömmlichen Oberflächenmessgerätes, bei dem immer nur eine Anlotung durchgeführt wird, leicht defokussiert ist, und der eine doppelte Vorrichtung zur Bildung von Wegen im Empfangsmodus (70, 70') enthält, um parallel und gleichzeitig am Ausgang der beiden Vorrichtungen zur Bildung der Wege die Signale der beiden Anlotlinien A und B bereitzustellen, die durch den Abstand e voneinander getrennt sind.

4. Verfahren zur Messung der Elastizität $\gamma$ einer Arterie unter der Wirkung des Blutdrucks P mit Hilfe eines Ultraschall-Echographen, der als Oberflächenmessgerät (M-mode) benutzt wird und mit Sende-/Empfangsmitteln mit einer Vorrichtung zur Bildung von Wegen im Empfangsmodus ausgestattet ist und in der Lage ist, die momentane Blutdurchflussmenge Q(t), die momentane Variation des Radius $\Delta r(t)$ und den mittleren Radius $r_0$ für die beiden dicht beieinanderliegenden Anlotlinien A und B, die die Arterie (2) entlang einer ihrer Meridianebenen durchqueren, zu liefern, für die Aktivierung des Ultraschall-Echographen ausgestattet mit der Messvorrichtung nach einem der Ansprüche 1 bis 3 - wobei die genannten Werte Q(t), $\Delta r(t)$ und $r_0$ in den genannten ersten (23) und zweiten (33) Speichermitteln gespeichert werden - bestehend aus den folgenden Schritten:

a) ausgehend von einer der beiden Funktionen Q(t), Q'(t) die Berechnung des Mittelwertes $Q_0$ und der Ableitung $\frac{\partial Q(t)}{\partial t}$ dieser Funktion;
b) ausgehend von den Momentanwerten $\Delta r(t)$, $\Delta r'(t)$ für die Linien A und B die Berechnung des Momentanwertes:

$$\delta r(t) = \Delta r(t) - \Delta r'(t)$$

c) ausgehend von der Methode der kleinsten Quadrate angewendet auf eine Herzperiode mit der Gleichung:

$$\gamma\left[RQ(t) + L\frac{\partial Q(t)}{\partial t} + \frac{\Delta P}{\Delta x}\right] = -\frac{\partial r(t)}{\partial x}$$

in der folgendes gilt:

$$L = \frac{\rho}{\Pi r_0^2} \quad \text{und} \quad \frac{\partial r(t)}{\partial x} = \frac{\delta r(t)}{e}$$

die Bestimmung der Mittelwerte der Elastizität $\gamma$, des hydrodynamischen Widerstands R und des Druckgradienten $\Delta P/\Delta x$, also von $\gamma_0$ , $R_0$ bzw. $R_0 Q_0$.

5. Verfahren zur Messung der Elastizität $\gamma$ einer Arterie nach Anspruch 4, <u>dadurch gekennzeichnet</u>, dass für die stückweise Bestimmung der Funktionen $\gamma(t)$ und R(t) über mindestens eine Herzperiode erneut die Methode der kleinsten Quadrate angewendet wird, und zwar durch aufeinanderfolgende Iterationen für die komplementären Abschnitte der Herzperiode mit ansteigender Reihenfolge von einer Iteration zur nächsten, wobei für die erste Iteration zwei halbe Herzperioden (dann 4, 8 ....., Abschnitte der Perioden für die nächsten Iterationen) genommen werden und die folgende Gleichung gilt:

$$\gamma_0(1 + g'(t))\left[R_0(1+R'(t))Q(t)+L\frac{\partial Q}{\partial t} + R_0 Q_0\right] = -\frac{\delta r(t)}{e}$$

wodurch man für $\gamma$ und R Funktionen erhält, die von einigen Abtastwerten während der Dauer der Herzperiode erfasst wurden.

**6.** Verfahren zur Messung des Drucks $P(t) = P_0 + p(t)$ im Inneren einer Arterie (2), mit dem der Wert von r(t) für eine der Anlotungen A oder B ausgehend von den Werten $r_0$ und $\Delta r(t)$ in Übereinstimmung mit Anspruch 5 bestimmt wird, <u>dadurch gekennzeichnet,</u> dass der variable Teil p(t) des Drucks durch die zeitliche Integration der Funktion der partiellen Ableitungen $P_1$ ermittelt wird:

$$P_1(t) = \int_t \frac{\partial P}{\partial t} dt = \int_t \frac{1}{\gamma(t)} \frac{\partial r}{\partial t} dt$$

gefolgt von der Unterdrückung der kontinuierlichen Komponente $P_c$ von $P_1(t)$:

$$p(t) = P_1(t) - P_c$$

und dass der feste Teil $P_0$ (zu p(t) hinzuzufügen) durch die Anwendung der Methode der kleinsten Quadrate auf die folgende Gleichung bestimmt wird:

$$\frac{\partial P}{\partial t} = \frac{1}{r}\frac{\partial T}{\partial t} - \frac{1}{r^2}\frac{\partial r}{\partial t}T$$

in der : $r = r(t)$ und $T = 1(t) = r(t) P(t)$,
wobei T(t) die im physikalischen Sinn durch das Blut auf den Umfang der Arterie ausgeübte Spannung ist, von der man in erster Näherung folgendes ableitet:

$$T_0 = \frac{1}{\gamma_0}\frac{\left\langle r\frac{\partial r}{\partial t}\right\rangle\left\langle\frac{1}{r}\frac{\partial r}{\partial t}\right\rangle - \left\langle\left(\frac{\partial r}{\partial t}\right)^2\right\rangle}{\left\langle\left(\frac{1}{r}\frac{\partial r}{\partial t}\right)^2\right\rangle - \left\langle\frac{1}{r}\frac{\partial r}{\partial t}\right\rangle^2}$$

wobei die Klammern ⟨ ⟩ den Mittelwert der eingeklammerten Funktion über eine Herzperiode angeben und :

$$P_0 = T_0/r_0 + P_e \text{ ist,}$$

wobei $P_e$ der konstante Druck außerhalb der Arterie ist.

FIG. 1

FIG. 2

FIG. 3

FIG. 4